# EUROPEAN PATENT APPLICATION

(11) **EP 4 817 611 A1**
(43) Date of publication of application: **07.10.2026**
(21) Application number: 24897673.0
(22) Date of filing: 29.11.2024
(51) Int. Cl.: A61K 31/343, A61K 31/381, A61K 47/04, A61K 47/12, A61K 47/18, A61K 47/26, A61P 1/16, A61P 3/06

(54) **PHARMACEUTICAL COMPOSITION**

(30) Priority: 01.12.2023 JP 2023203898
(71) Applicant: EA Pharma Co., Ltd., Tokyo 104-0042 (JP)
(72) Inventor: SUGIURA Daisuke, Tokyo 104-0042 (JP); ARISAKA Harumi, Tokyo 104-0042 (JP); NAKAHARA Wataru, Tokyo 104-0042 (JP); HIRANO Satoshi, Tokyo 104-0042 (JP); KIDO Keisuke, Tokyo 104-0042 (JP)
(74) Representative: Mewburn Ellis LLP
(86) International application number: PCT/JP2024/042254
(87) International publication number: WO 2025/115989

(57) **Abstract**

A pharmaceutical composition comprising an active ingredient having an ester bond and an acidic substance.

## Description

### Technical Field

The present disclosure relates to a pharmaceutical composition.

The present application claims priority based on Japanese Patent Application No. 2023-203898 filed in Japan on December 1, 2023, the contents of which are incorporated herein.

### Background Art

In order to provide effective and safe medicaments, it is necessary to ensure their stability. If there is a problem with the stability of medicaments, there may be problems, for example, that the active ingredients decompose and their contents decrease, resulting in a decrease in efficacy, and that the decomposition products of the active ingredients have a negative impact on safety.

For the purpose of improving stability, Patent Literature 1 discloses a "pharmaceutical composition comprising (R)-2-(4-bromo-2-fluorobenzyl)-1,2,3,4-tetrahydropyrrolo[1,2-a]pyrazine-4-spiro-3'-pyrrolidine-1,2',3,5'-tetraone as an active ingredient and, as a stabilizer, at least one acidic substance that has an acidity higher than that of the active ingredient compound." Patent Literature 2 also discloses similar contents as Patent Literature 1.

For the purpose of improving stability, Patent Literature 3 discloses a pharmaceutical composition comprising an alkaloid ether oxime salt and an acidic filler.

### Citation List

### Patent Literature

Patent Literature 1: International Publication No. WO 1999/20276
Patent Literature 2: International Publication No. WO 1999/20277
Patent Literature 3: International Publication No. WO 1994/07493

### Summary of Invention

### Technical Problem

The present disclosure relates to a pharmaceutical composition whose active ingredient having an ester bond has improved stability.

### Solution to Problem

The use of an acidic substance makes it possible to improve the stability of an active ingredient having an ester bond.

The present disclosure includes the following embodiments.
[1] A pharmaceutical composition comprising:
   an active ingredient having an ester bond; and
   an acidic substance.
[2] The pharmaceutical composition according to [1], wherein the active ingredient is a compound represented by the following formula (1) or a pharmaceutically acceptable salt thereof: 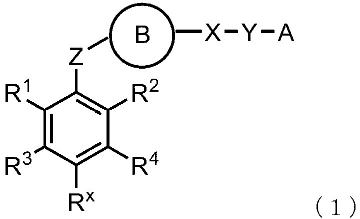 [wherein
   R¹, R², R³, and R⁴ are each independently a hydrogen atom, a nitro group, a halogeno group, a cyano group, a hydroxyl group, a lower alkyl group, or a lower alkoxyl group,
   R^{x} is an amidino group or a guanidino group,
   Z is -O(C=O)- or -(C=O)O-,
   B is an optionally substituted heterocycle,
   X is an optionally substituted lower alkylene group, an optionally substituted lower alkenylene group, or an optionally substituted lower alkynylene group,
   Y is a carbonyl group, and
   A is selected from the following groups:
      · -OR⁵ (wherein R⁵ is a hydrogen atom or a lower alkyl group); or
      · the following group (1A-1): 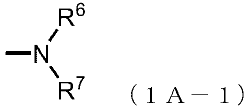
      (wherein
   R⁶ and R⁷ are each independently a hydrogen atom or an optionally substituted lower alkyl group, or
   R⁶ and R⁷, together with the nitrogen atom to which they are attached, optionally form an optionally substituted cyclic amino group.)]
[3] The pharmaceutical composition according to [1] or [2], wherein the active ingredient is a compound represented by the following formula (2) or (3) or a pharmaceutically acceptable salt thereof: 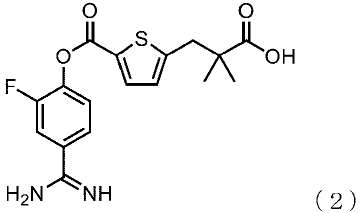 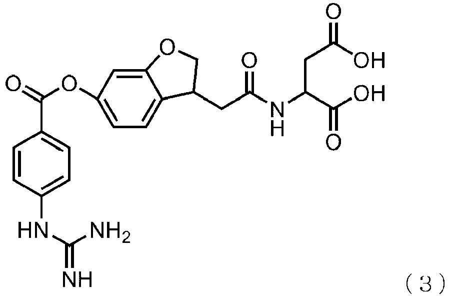
[4] The pharmaceutical composition according to [2] or [3], wherein the pharmaceutically acceptable salt is hydrochloride.
[5] The pharmaceutical composition according to any one of [1] to [4], wherein the acidic substance has a pKa of 1.0 to 6.0.
[6] The pharmaceutical composition according to any one of [1] to [5], wherein the acidic substance comprises at least one selected from the group consisting of fumaric acid, succinic acid, adipic acid, aspartic acid, glycine, benzoic acid, citric acid, tartaric acid, lactic acid, maleic acid, and malic acid.
[7] The pharmaceutical composition according to any one of [1] to [6], wherein the acidic substance comprises at least one selected from the group consisting of fumaric acid, succinic acid, adipic acid, aspartic acid, glycine, and benzoic acid.
[8] The pharmaceutical composition according to any one of [1] to [7], wherein the acidic substance comprises fumaric acid and/or succinic acid.
[9] The pharmaceutical composition according to any one of [1] to [8], wherein the acidic substance comprises fumaric acid and succinic acid.
[10] The pharmaceutical composition according to any one of [1] to [9], further comprising an filler.
[11] The pharmaceutical composition according to [10], wherein the filler is coated with the acidic substance.
[12] The pharmaceutical composition according to [10] or [11], wherein the filler and the active ingredient are coated with the acidic substance.

### Advantageous Effect of Invention

It is possible to improve the stability of an active ingredient having an ester bond.

### Brief Description of Drawings

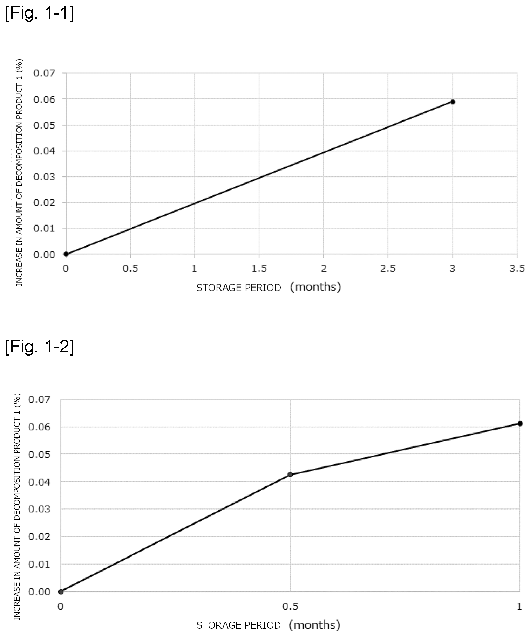

[Figure 1-1] Figure 1-1 shows the results of a stability test under airtight conditions at 40°C for the pharmaceutical composition of Comparative Example 1 (without an acidic substance).
[Figure 1-2] Figure 1-2 shows the results of a stability test under airtight conditions at 60°C for the pharmaceutical composition of Comparative Example 2 (without an acidic substance).
[Figure 2-1] Figure 2-1 shows the results of a stability test under airtight conditions at 40°C for the pharmaceutical compositions of Example 1-1 (production method A; formulation 1-1), Example 1-2 (production method B; formulation 1-1), and Example 1-3 (production method C; formulation 1-1).
[Figure 2-2] Figure 2-2 shows the results of a stability test under airtight conditions at 40°C for the pharmaceutical compositions of Example 1-4 (production method A; formulation 1-2), Example 1-5 (production method B; formulation 1-2), and Example 1-6 (production method C; formulation 1-2).
[Figure 3] Figure 3 shows the results of a stability test under airtight conditions at 40°C for the pharmaceutical compositions of Example 2-1 (production method A; formulation 2-1), Example 2-2 (production method C; formulation 2-1), and Example 2-3 (production method D; formulation 2-1).
[Figure 4] Figure 4 shows the results of a stability test under airtight conditions at 40°C for the pharmaceutical compositions of Example 3-1 (production method C; formulation 3-1), Example 3-2 (production method C; formulation 3-2), and Example 3-3 (production method C; formulation 3-3).
[Figure 5] Figure 5 shows the results of a stability test under airtight conditions at 40°C for the pharmaceutical composition of Example 5 (production method C; formulation 3-3) with scaled-up production.

### Description of Embodiments

Embodiments of the present disclosure will be described in detail below; however, the present disclosure is not limited thereto, and various modifications are possible without departing from the gist thereof.

### <Definition>

In the present specification, "comprise" and "contain" mean that, in addition to the elements explicitly stated to be contained, other elements may also be contained.

In the present specification, the "lower alkyl group" refers to a linear, branched, or cyclic alkyl group having 1 to 6 carbon atoms.

In the present specification, the "lower alkylene group" refers to a linear, branched, or cyclic alkylene group having 1 to 6 carbon atoms.

In the present specification, the "lower alkenylene group" refers to a linear or branched alkenylene group having 2 to 6 carbon atoms.

In the present specification, the "lower alkynylene group" refers to a linear or branched lower alkynylene group having 2 to 6 carbon atoms.

In the present specification, the "lower alkoxyl group" refers to an alkoxyl group having a lower alkyl group.

In the present specification, the "lower alkylthio group" refers to an alkylthio group having a lower alkyl group.

In the present specification, the "lower alkoxycarbonyl group" refers to a carbonyl group having a lower alkoxyl group.

### <Pharmaceutical Composition>

An embodiment of the present disclosure relates to a pharmaceutical composition comprising an active ingredient having an ester bond (hereinafter also referred to simply as "the active ingredient") and an acidic substance.

In the pharmaceutical composition according to the present embodiment, the acidic substance can suppress the decomposition of the active ingredient due to the cleavage of the ester bond.

### [Active Ingredient]

The pharmaceutical composition according to the present embodiment comprises an active ingredient having an ester bond. The active ingredient is preferably a compound represented by the following formula (1) or a pharmaceutically acceptable salt:

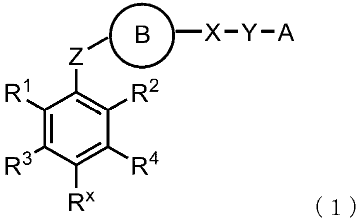

### (R¹ and R²)

R¹ and R² in the formula (1) are each independently a hydrogen atom, a nitro group, a halogeno group, a cyano group, a hydroxyl group, a lower alkyl group, or a lower alkoxyl group.

R¹ and R² in the formula (1) are preferably hydrogen atoms or halogeno groups, and more preferably hydrogen atoms or fluorine.

### (R³ and R⁴)

R³ and R⁴ in the formula (1) are each independently a hydrogen atom, a nitro group, a halogeno group, a cyano group, a hydroxyl group, a lower alkyl group, or a lower alkoxyl group.

R³ and R⁴ in the formula (1) are preferably hydrogen atoms or halogeno groups, and more preferably hydrogen atoms.

### (R^{x})

R^{x} in the formula (1) is an amidino group or a guanidino group, and preferably an amidino group.

### (Z)

Z in the formula (1) is -O(C=O)- or -(C=O)O-, and preferably -O(C=O)-.

In -O(C=O)-, the carbonyl group "(C=O)-" binds to B in the formula (1). In - (C=O)O-, the oxygen atom "O-" binds to B in the formula (1).

### (B)

B in the formula (1) is an optionally substituted heterocycle, preferably an optionally substituted 5- to 10-membered monocyclic or bicyclic heterocycle, more preferably an optionally substituted 5- to 10-membered monocyclic or bicyclic aromatic heterocycle, even more preferably an optionally substituted 5- or 6-membered monocyclic aromatic heterocycle, and particularly preferably a thiophene ring. A dihydrobenzofuran ring, which is a bicyclic heterocycle, is also particularly preferred.

The number of heteroatoms contained in the heterocycle is preferably 1 to 3, more preferably 1 or 2, and even more preferably 1.

The heteroatom is preferably at least one selected from the group consisting of an oxygen atom, a sulfur atom, and a nitrogen atom, more preferably an oxygen atom and/or a sulfur atom, and even more preferably a sulfur atom.

Examples of the substituent in B include a halogeno group and a lower alkyl group.

### (X)

X in the formula (1) is an optionally substituted lower alkylene group, an optionally substituted lower alkenylene group, or an optionally substituted lower alkynylene group.

X in the formula (1) is preferably an optionally substituted lower alkylene group, more preferably an optionally substituted alkylene group having 1 to 4 carbon atoms, even more preferably an optionally substituted branched alkylene group having 4 carbon atoms, and particularly preferably -CH₂-C(CH₃)₂-.

In -CH₂-C(CH₃)₂-, the carbon atoms substituted with two methyl groups are preferably bonded to Y in the formula (1).

Examples of the substituent in X include a halogeno group and a lower alkyl group.

### (Y)

Y in the formula (1) is a carbonyl group.

### (A)

A in the formula (1) is selected from the following groups:
· -OR⁵ (wherein R⁵ is a hydrogen atom or a lower alkyl group); or
· the following group (1A-1): 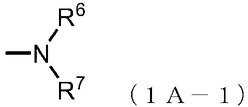
(wherein
R⁶ and R⁷ are each independently a hydrogen atom or an optionally substituted lower alkyl group, or
R⁶ and R⁷, together with the nitrogen atom to which they are attached, optionally form an optionally substituted cyclic amino group.)

A in the formula (1) is preferably -OH or the above group (1A-1) (wherein R⁶ is a hydrogen atom, and R⁷ is an optionally substituted lower alkyl group, preferably an optionally substituted alkyl group having 1 to 3 carbon atoms, more preferably an alkyl group having 1 to 3 carbon atoms substituted with a carboxyl group, even more preferably an ethyl group substituted with a carboxyl group, and particularly preferably -CH(COOH)-CH₂(COOH)), and more preferably -OH.

Examples of the substituents in R⁶ and R⁷ include a hydroxyl group, a thiol group, an amino group, a guanidino group, a carboxyl group, a lower alkylthio group, a lower alkoxycarbonyl group, a carbamoyl group, an optionally substituted aryl group, and an optionally substituted heterocyclic group.

Examples of the substituent in the cyclic amino group formed by bonding of R⁶ and R⁷ include a hydroxy group and a carboxyl group.

The active ingredient in the present embodiment is preferably a compound represented by the following formula (2) or (3) or a pharmaceutically acceptable salt thereof:

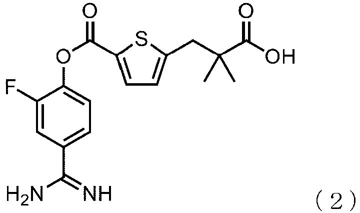

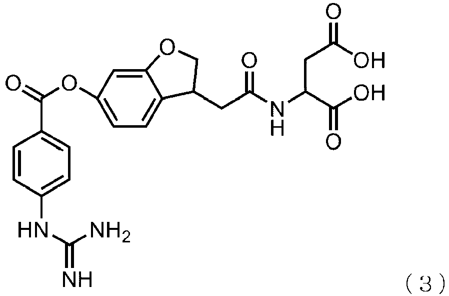

The pharmaceutically acceptable salt as the active ingredient in the present embodiment is not particularly limited as long as it can be used as a medicament, and examples thereof include inorganic acid salts, such as hydrochloride, sulfate, nitrate, hydrobromide, and phosphate; and organic acid salts, such as fumarate, maleate, malate, tartrate, citrate, succinate, methanesulfonate, p-toluenesulfonate, lactate, acetate, and palmitate. The pharmaceutically acceptable salt is preferably hydrochloride.

The active ingredient in the present embodiment may form a solvate, such as a hydrate. In the present specification, solvates are included in the compounds represented by the above formulas or pharmaceutically acceptable salts thereof.

When stereoisomers (e.g., enantiomers and diastereomers) are present in the active ingredient in the present embodiment, the individual stereoisomers and mixtures thereof (e.g., racemates) are included in the compounds represented by the above formulas or pharmaceutically acceptable salts thereof.

The active ingredient in the present embodiment can be synthesized with reference to a known method (e.g., the method described in International Publication No. WO 2011/071048, International Publication No. WO 2015/137407, or International Publication No. WO 2015/137408).

The active ingredient is preferably coated with an acidic substance, described later. Part of the surface of the active ingredient may be coated with the acidic substance, or the entire surface of the active ingredient may be coated with the acidic substance. When the active ingredient is coated with the acidic substance, the decomposition of the active ingredient can be further suppressed.

The amount of the active ingredient may be a therapeutically effective amount. The therapeutically effective amount may be appropriately determined depending on, for example, the type and progression of the disease, and the weight and condition of the patient.

### [Acidic Substance]

The pharmaceutical composition according to the present embodiment comprises an acidic substance. The acidic substance can suppress the decomposition of the active ingredient.

The acid dissociation constant (pKa) of the acidic substance is preferably 1.0 to 6.0, more preferably 1.5 to 5.5, even more preferably 2.0 to 5.0, particularly preferably 2.5 to 4.5, and most preferably 3.0 to 4.5. When an acidic substance having pKa in such a range is used, the decomposition of the active ingredient can be further suppressed.

If the acidic substance has multiple pKa values, the first-stage acid dissociation constant (pKa₁) is used as the reference.

In the present specification, the acid dissociation constant (pKa) of the acidic substance may be an experimentally measured acid dissociation constant, or may be an acid dissociation constant recorded in a known database, such as the CAS database or the Chemical Book, or an acid dissociation constant estimated using a known program. In the present specification, pKa recorded in the Chemical Book, which is a known database, is given as an example of the acid dissociation constant of the acidic substance; however, a person skilled in the art would have been able to refer to this database or use a database or program having equivalent functions to recognize values recorded as acid dissociation constants of any acidic substances as the scope of the present disclosure. When measured experimentally, it is preferable to follow the measurement conditions described in the above database, including temperature and the like. If the pKa values obtained from databases, programs, and experiments do not match, the pKa value described in the Chemical Book takes precedence.

The acidic substance preferably comprises at least one selected from the group consisting of fumaric acid (pKa₁: 3.02, pKa₂: 4.38), succinic acid (pKa: 4.16), adipic acid (pKa: 4.43), aspartic acid (pKa: 1.99), glycine (pKa: 2.35), benzoic acid (pKa: 4.19), citric acid (pKa: 3.14), tartaric acid (pKa: 3.03), lactic acid (pKa: 3.86), maleic acid (pKa: 1.83), and malic acid (pKa: 3.4). These acidic substances also include their salts, acid anhydrides, and solvates.

The acidic substance more preferably comprises at least one selected from the group consisting of fumaric acid, succinic acid, adipic acid, aspartic acid, glycine, and benzoic acid, even more preferably fumaric acid and/or succinic acid, and particularly preferably fumaric acid and succinic acid.

The amount of the acidic substance based on the entire mass of the pharmaceutical composition is preferably 0.1 to 30 mass%, more preferably 0.2 to 10.0 mass%, even more preferably 0.3 to 5.0 mass%, and particularly preferably 0.5 to 3.0 mass%.

### [Filler]

The pharmaceutical composition according to the present embodiment may further comprise an filler. The use of an filler in combination with an acidic substance can facilitate handling during the production process and further efficiently suppress the decomposition of the active ingredient.

The type of filler is not particularly limited, and a pharmaceutically acceptable filler may be used. Examples of fillers include lactic acid hydrate, anhydrous lactose, croscarmellose sodium, carmellose, magnesium carbonate, mannitol, crystalline cellulose, anhydrous calcium hydrogen phosphate, methacrylic copolymers, calcium carbonate, magnesium oxide, and sodium chloride.

The filler is preferably coated with an acidic substance. In the present specification, "coating" includes covering the target surface and adhering to the target surface. In the pharmaceutical composition of the present embodiment, at least part of the surface of the filler may be coated with an acidic substance uniformly or non-uniformly, or the entire surface of the filler may be coated with an acidic substance uniformly or non-uniformly. The use of an filler coated with an acidic substance can increase the contact area between the active ingredient and the acidic substance, and further suppress the decomposition of the active ingredient.

The amount of the filler based on the total mass of the pharmaceutical composition is preferably 5 to 95 mass%, more preferably 10 to 90 mass%, and even more preferably 20 to 80 mass%.

### [Other Ingredients]

The pharmaceutical composition according to the present embodiment may further contain other ingredients as long as the technical effects of the present disclosure are not impaired. Although other ingredients vary depending on the dosage form and the like, examples thereof include glidants (e.g., silicon dioxide), disintegrants, surfactants, suspending agents, emulsifiers, preservatives, colorants, fragrances, sweeteners, flavorings, and thickeners. As the above other ingredients, known ingredients can be appropriately used singly or in combination of two or more.

### [Dosage Form]

The pharmaceutical composition according to the present embodiment can be administered orally or parenterally. Examples of dosage forms for oral administration include tablets, pills, granules, powders, capsules, syrups, emulsions, and suspensions. Examples of dosage forms for parenteral administration include injections, infusions, intravenous infusions, eye drops, and suppositories. Oral administration is preferred, with tablets, pills, granules, powders, or capsules being more preferred.

### [Use]

The pharmaceutical composition according to the present embodiment can be used for the treatment of fatty liver disease. The term "treatment" as used herein includes prevention of the onset of fatty liver disease, suppression of the progress of fatty liver disease, alleviation of the symptoms of fatty liver disease, and cure of fatty liver disease.

Specific examples of fatty liver disease include non-alcoholic fatty liver disease (NAFLD), non-alcoholic steatohepatitis (NASH), metabolic-associated fatty liver disease (MAFLD or MASLD), metabolic-associated steatohepatitis (MASH), and fatty liver.

### <Method for Producing Pharmaceutical Composition>

Although the method for producing the pharmaceutical composition described above is not particularly limited, examples thereof include the following production methods A to D. In order to further suppress the decomposition of the active ingredient, the production method C or D is preferred. According to the production method C, when an filler is coated with an acidic substance, the decomposition of the active ingredient can be further suppressed. According to the production method D, when an active ingredient and an filler are coated with an acidic substance, the decomposition of the active ingredient can be further suppressed.

### [Production Method A]

A method for producing a pharmaceutical composition, comprising:
a mixing step: mixing (preferably physically mixing) constituent components of the pharmaceutical composition.

### [Production Method B]

A method for producing a pharmaceutical composition, comprising:
a grinding step: grinding (preferably jet-milling) an acidic substance and an filler together, and
a mixing step: mixing (preferably physically mixing) the ground acidic substance and filler with the remaining components of the pharmaceutical composition.

### [Production Method C]

A method for producing a pharmaceutical composition, comprising:
a coating step: spraying an acidic substance to an filler to obtain the filler coated with the acidic substance, and
a mixing step: mixing (preferably physically mixing) the filler coated with the acidic substance with the remaining components of the pharmaceutical composition.

### [Production Method D]

A method for producing a pharmaceutical composition, comprising:
a coating step: spraying an acidic substance to an active ingredient and an filler to obtain the active ingredient and filler coated with the acidic substance, and
a mixing step: mixing (preferably physically mixing) the active ingredient and filler coated with the acidic substance with the remaining components of the pharmaceutical composition.

In the production method C, it is preferable to perform granulation (preferably fluidized bed granulation) of an acidic substance and an filler simultaneously with the coating step.

In the production method D, it is preferable to perform granulation (preferably fluidized bed granulation) of an acidic substance, an active ingredient, and an filler simultaneously with the coating step.

In the production method C or D, when an acidic substance is sprayed in the coating step, the acidic substance can be dissolved in a suitable medium and sprayed. The suitable medium is not particularly limited as long as it can dissolve the acidic substance, but is preferably water or a lower alcohol-containing solution, such as ethanol.

### <Medicament>

An embodiment of the present disclosure relates to a medicament in which the pharmaceutical composition described above in an amount effective for the treatment of the fatty liver disease described above is stored in an appropriate container. Storage in an appropriate container makes it less susceptible to the influence of the external environment of the container and further suppresses the decomposition of the active ingredient.

Appropriate containers are not particularly limited and may be any containers that can be used to store medicaments. Preferred are containers that can maintain an appropriate airtight environment therein. Examples of such containers include glass or plastic vials, ampoules, or bottles, PTP sheets, aluminum packaging, and the like. The inside of the container may be replaced with an inert gas or the like, if necessary.

### Examples

The present disclosure will be described in more detail below using Examples and Comparative Examples; however, the technical scope of the present disclosure is not limited thereto.

### <Preparation of Pharmaceutical Compositions>

Pharmaceutical compositions were prepared according to the following production methods A to D and the formulations in the following tables.

### [Production Method A]

Components of the pharmaceutical composition were physically mixed.

### [Production Method B]

An acidic substance and an filler were jet-milled together, and the resultant was then physically mixed with the remaining components of the pharmaceutical composition.

### [Production Method C]

An acidic substance was sprayed to an filler for fluidized bed granulation, and the resultant was then physically mixed with the remaining components of the pharmaceutical composition.

### [Production Method D]

An acidic substance was sprayed to an active ingredient and an filler for fluidized bed granulation, and the resultant was then physically mixed with the remaining components of the pharmaceutical composition.

### <Stability Test>

When the hydrochloride of the compound represented by the formula (2) (hereinafter referred to as "Compound (2) HCl") is decomposed, the following decomposition products 1 and 2 are produced.

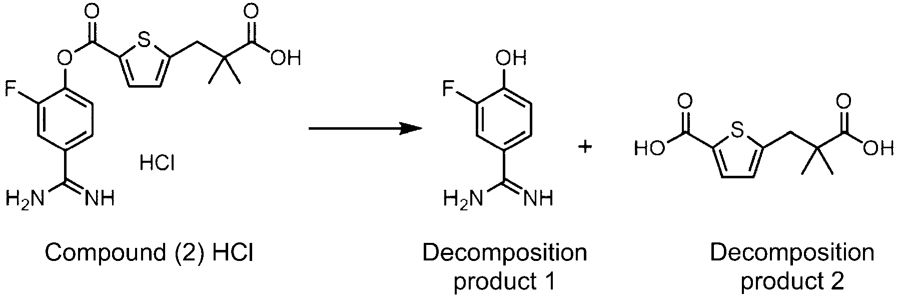

The amount of the decomposition product 1 produced was measured over time by HPLC to evaluate the stability of the pharmaceutical composition. The HPLC analysis conditions are as follows. When airtight conditions were employed in the stability test, aluminum packaging was used.

### [HPLC Conditions]

Detector: UV absorption photometer (measurement wavelength: 256 nm)
Column: InertSustain AQ-C₁₈ HP 3 µm, 3.0 × 150 mm (reverse-phase C₁₈ column)
Column temperature: a constant temperature around 25°C
Mobile phase A: 50 mmol/L ammonium formate buffer solution (pH: 4)
Mobile phase B: 50 mmol/L ammonium formate buffer solution (pH: 4) : acetonitrile = 1:4
Flow rate: 1.0 ml/min

### Gradient conditions

Time: 0 → 20
Mobile phase A (%): 95 → 0
Mobile phase B (%): 5 → 100

### [Comparative Example 1]

A pharmaceutical composition was prepared according to the formulation in Table 1 and the production method A, and a stability test was performed under airtight conditions (with a desiccant) at 40°C. The results are shown in Figure 1-1.

### [Comparative Example 2]

A pharmaceutical composition was prepared according to the formulation in Table 1 and the production method A, and a stability test was performed under airtight conditions (with a desiccant) at 60°C. The results are shown in Figure 1-2. [Table 1]

**Table 1**

| | | Comparative Formulation 1 |
|---|---|---|
| | | Blending amount (mg/cap) |
| Active ingredient | Compound (2) HCl | 55 |
| Filler | D-mannitol | 199.8 |
| Lubricant | Magnesium stearate | 2.6 |
| Glidant | Silicon dioxide | 2.6 |
| Acidic substance | Fumaric acid | - |
| | Succinic acid | - |
| Total | | 260 |

### [Examples 1-1 to 1-6: Examination 1 of Production Methods]

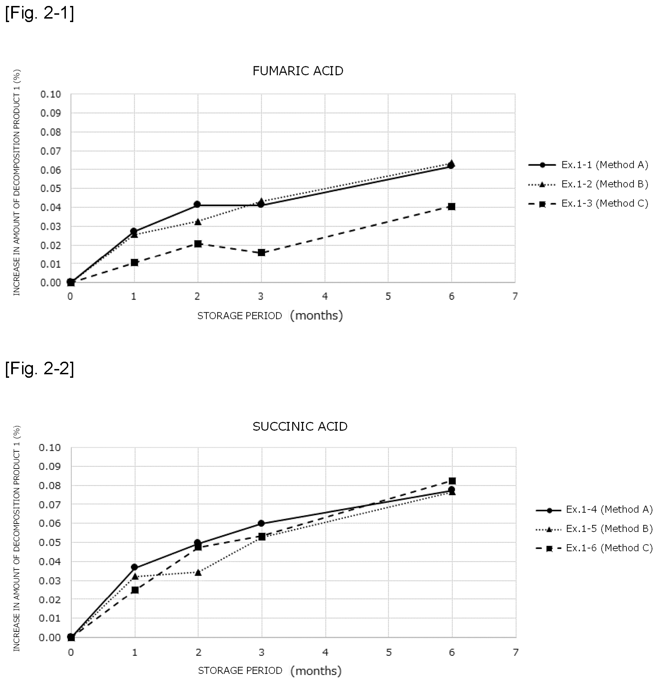

Pharmaceutical compositions were prepared according to Tables 2-1 and 2-2, and a stability test was performed under airtight conditions (with a desiccant) at 40°C. The results of Examples 1-1 to 1-3 are shown in Figure 2-1. The results of Examples 1-4 to 1-6 are shown in Figure 2-2. Compared to Comparative Example 1, the stability was improved in all of Examples 1-1 to 1-3 regardless of the production method, and the stability was maintained or improved in Examples 1-4 to 1-6.

[Table 2-1]

**Table 2-1**

| | | Formulation 1-1 | Formulation 1-2 |
|---|---|---|---|
| | | Blending amount (mg/cap) | |
| Active ingredient | Compound (2) HCl | 55 | 55 |
| Filler | D-mannitol | 195.8 | 195.8 |
| Lubricant | Magnesium stearate | 2.6 | 2.6 |
| Glidant | Silicon dioxide | 2.6 | 2.6 |
| Acidic substance | Fumaric acid | 4 | - |
| | Succinic acid | - | 4 |
| Total | | 260 | 260 |

[Table 2-2]

**Table 2-2**

| | Production method | Formulation |
|---|---|---|
| Example 1-1 | A | 1-1 |
| Example 1-2 | B | 1-1 |
| Example 1-3 | C | 1-1 |
| Example 1-4 | A | 1-2 |
| Example 1-5 | B | 1-2 |
| Example 1-6 | C | 1-2 |

### [Examples 2-1 to 2-3: Examination 2 of Production Methods]

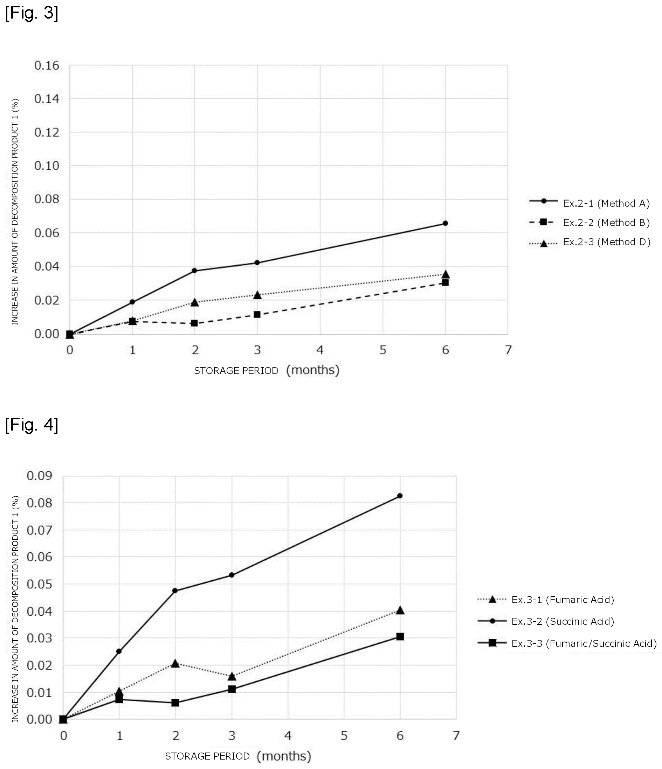

Pharmaceutical compositions were prepared according to Tables 3-1 and 3-2, and a stability test was performed under airtight conditions (with a desiccant) at 40°C. The results are shown in Figure 3. The pharmaceutical compositions prepared by any production methods showed the same or better stability improvement effect compared to Comparative Example 1.

[Table 3-1]

**Table 3-1**

| | | Formulation 2-1 |
|---|---|---|
| | | Blending amount (mg/cap) |
| Active ingredient | Compound (2) HCl | 55 |
| Filler | D-mannitol | 191.8 |
| Lubricant | Magnesium stearate | 2.6 |
| Glidant | Silicon dioxide | 2.6 |
| Acidic substance | Fumaric acid | 4 |
| | Succinic acid | 4 |
| Total | | 260 |

[Table 3-2]

**Table 3-2**

| | Production method | Formulation |
|---|---|---|
| Example 2-1 | A | 2-1 |
| Example 2-2 | C | 2-1 |
| Example 2-3 | D | 2-1 |

### [Examples 3-1 to 3-3: Examination 1 of Acidic Substances]

Pharmaceutical compositions were prepared according to Tables 4-1 and 4-2, and a stability test was performed under airtight conditions (with a desiccant) at 40°C. The results are shown in Figure 4. Example 3-3, which used two acidic substances in combination, showed further improved stability compared to Examples 3-1 and 3-2, each of which used a single acidic substance.

[Table 4-1]

**Table 4-1**

| | | Formulation 3-1 | Formulation 3-2 | Formulation 3-3 |
|---|---|---|---|---|
| | | Blending amount (mg/cap) | | |
| Active ingredient | Compound (2) HCl | 55 | 55 | 55 |
| Filler | D-mannitol | 195.8 | 195.8 | 191.8 |
| Lubricant | Magnesium stearate | 2.6 | 2.6 | 2.6 |
| Glidant | Silicon dioxide | 2.6 | 2.6 | 2.6 |
| Acidic substance | Fumaric acid | 4 | - | 4 |
| | Succinic acid | - | 4 | 4 |
| Total | | 260 | 260 | 260 |

[Table 4-2]

**Table 4-2**

| | Production method | Formulation |
|---|---|---|
| Example 3-1 | C | 3-1 |
| Example 3-2 | C | 3-2 |
| Example 3-3 | C | 3-3 |

### [Examples 4-1 to 4-16: Examination 2 of Acidic Substances]

The formulation in Table 5-1 (the types of acidic substances are listed in Table 5-2) was mixed in a mortar to prepare a pharmaceutical composition, which was then sealed in a glass vial, and a one-month stability test was performed under three storage conditions (airtight conditions at 4°C, airtight conditions at 60°C, and open conditions at 60°C). As a reference example, a stability test was also performed on the active ingredient alone (without an acidic substance). The results are shown in Table 5-2. The stabilizing effect of the compound (2) was also exhibited when using acidic substances other than the acidic substances used in Examples 1-1 to 3-3.

[Table 5-1]

**Table 5-1**

| | | Formulation 4-1 |
|---|---|---|
| | | Blending amount (mg/vial) |
| Active ingredient | Compound (2) HCl | 62.5 |
| Acidic substance | Various types | 62.5 |
| Total | | 125 |

**[Table 5-2]**

| Table 5-2: One-month stability test | | | | |
|---|---|---|---|---|
| | Acidic substance | Content of decomposition product 1 (%) (4°C, airtight) | Content of decomposition product 1 (%) (60°C, airtight) | Content of decomposition product 1 (%) (60°C, open, 75% RH) |
| Reference example | None | 0.03 | 0.05 | 0.10 |
| Example 4-1 | Adipic acid | 0.03 | 0.04 | 0.07 |
| Example 4-2 | L-aspartic acid | 0.04 | 0.07 | 0.12 |
| Example 4-3 | Glycine | 0.07 | 0.09 | 0.12 |
| Example 4-4 | Benzoic acid | 0.02 | 0.03 | 0.07 |
| Example 4-5 | Citric acid hydrate | 0.03 | 1.38 | 6.72 |
| Example 4-6 | Sodium dihydrogen citrate | 0.05 | 0.28 | 4.30 |
| Example 4-7 | Tartaric acid | 0.03 | 0.24 | 5.92 |
| Example 4-8 | D-tartaric acid | 0.02 | 0.17 | 5.02 |
| Example 4-9 | Lactic acid | 0.03 | 1.87 | 9.00 |
| Example 4-10 | Fumaric acid | 0.02 | 0.03 | 0.07 |
| Example 4-11 | Monosodium fumarate | 0.12 | 0.15 | 0.16 |
| Example 4-12 | Succinic acid | 0.03 | 0.06 | 0.08 |
| Example 4-13 | Maleic acid | 0.02 | 0.03 | 0.83 |
| Example 4-14 | Anhydrous citric acid | 0.02 | 0.30 | 6.87 |
| Example 4-15 | DL-malic acid | 0.02 | 0.07 | 6.29 |

### [Example 5: Examination 3 of Production Methods]

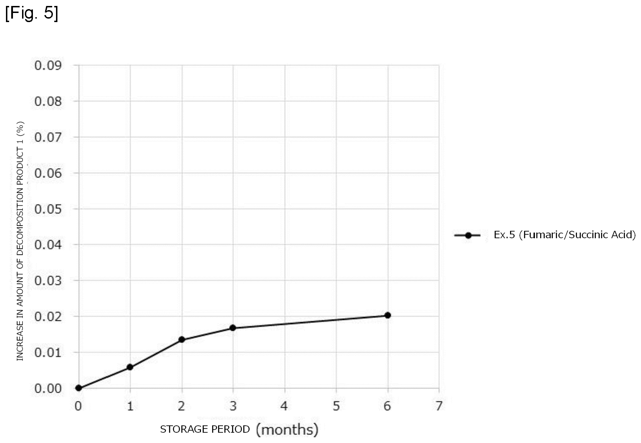

A pharmaceutical composition was prepared according to the production method C. 16.0 kg of a spray liquid (50% ethanol aqueous solution) containing 400 g of fumaric acid and 400 g of succinic acid as acidic substances was prepared and sprayed to 19.18 kg of an filler (D-mannitol) to perform fluidized bed granulation. The resulting granules were physically mixed with the remaining components of the pharmaceutical composition in the same manner as in the formulation 3-3. Figure 5 shows the results of a stability test conducted under airtight conditions (with a desiccant) at 40°C. Even when the coating step in the production method C was scaled up for production, the stabilizing effect of the compound (2) when using acidic substances was at least equivalent to that of Example 3-3.

## Claims

1. A pharmaceutical composition comprising:
an active ingredient having an ester bond; and
an acidic substance.

2. The pharmaceutical composition according to claim 1, wherein the active ingredient is a compound represented by the following formula (1) or a pharmaceutically acceptable salt thereof: 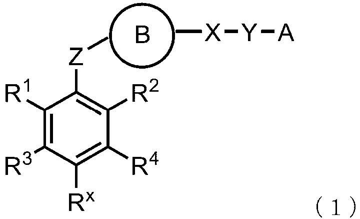 [wherein
R¹, R², R³, and R⁴ are each independently a hydrogen atom, a nitro group, a halogeno group, a cyano group, a hydroxyl group, a lower alkyl group, or a lower alkoxyl group,
R^{x} is an amidino group or a guanidino group,
Z is -O(C=O)- or -(C=O)O-,
B is an optionally substituted heterocycle,
X is an optionally substituted lower alkylene group, an optionally substituted lower alkenylene group, or an optionally substituted lower alkynylene group,
Y is a carbonyl group, and
A is selected from the following groups:
· -OR⁵ (wherein R⁵ is a hydrogen atom or a lower alkyl group); or
· the following group (1A-1): 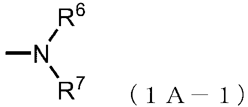
(wherein
R⁶ and R⁷ are each independently a hydrogen atom or an optionally substituted lower alkyl group, or
R⁶ and R⁷, together with the nitrogen atom to which they are attached, optionally form an optionally substituted cyclic amino group.)]

3. The pharmaceutical composition according to claim 1 or 2, wherein the active ingredient is a compound represented by the following formula (2) or (3) or a pharmaceutically acceptable salt thereof:

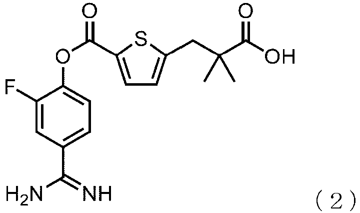

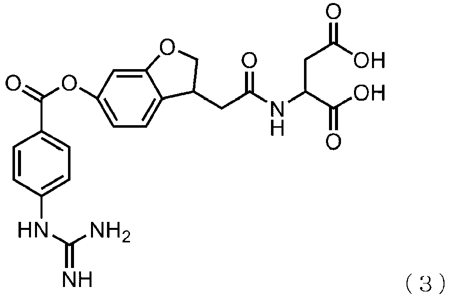

4. The pharmaceutical composition according to claim 3, wherein the pharmaceutically acceptable salt is hydrochloride.

5. The pharmaceutical composition according to claim 1 or 2, wherein the acidic substance has a pKa of 1.0 to 6.0.

6. The pharmaceutical composition according to claim 1 or 2, wherein the acidic substance comprises at least one selected from the group consisting of fumaric acid, succinic acid, adipic acid, aspartic acid, glycine, benzoic acid, citric acid, tartaric acid, lactic acid, maleic acid, and malic acid.

7. The pharmaceutical composition according to claim 6, wherein the acidic substance comprises at least one selected from the group consisting of fumaric acid, succinic acid, adipic acid, aspartic acid, glycine, and benzoic acid.

8. The pharmaceutical composition according to claim 7, wherein the acidic substance comprises fumaric acid and/or succinic acid.

9. The pharmaceutical composition according to claim 8, wherein the acidic substance comprises fumaric acid and succinic acid.

10. The pharmaceutical composition according to claim 1 or 2, further comprising an filler.

11. The pharmaceutical composition according to claim 10, wherein the filler is coated with the acidic substance.

12. The pharmaceutical composition according to claim 10, wherein the filler and the active ingredient are coated with the acidic substance.
